# EUROPEAN PATENT APPLICATION

(11) **EP 1 767 646 A1**
(43) Date of publication of application: **28.03.2007**
(21) Application number: 05741379.1
(22) Date of filing: 17.05.2005
(51) Int. Cl.: C12Q 1/02, C12N 15/09, G01N 21/78, G01N 33/15, G01N 33/50, G01N 33/566, C07K 7/06, C07K 7/08, C07K 14/705, C07K 19/00

(54) **PROBE FOR DETECTION/DETERMINATION OF INOSITOL-1,4,5-TRIPHOSPHORIC ACID AND METHOD OF DETECTING/DETERMINING INOSITOL-1,4,5-TRIPHOSPHORIC ACID WITH THE SAME**

(30) Priority: 21.05.2004 JP 2004152484
(71) Applicant: Japan Science and Technology Agency, Kawaguchi-shi, Saitama 332-0012 (JP)
(72) Inventor: UMEZAWA, Yoshio, Shinjuku-ku, Tokyo 162-0063 (JP); SATO, Moritoshi, Bunkyo-ku, Tokyo 113-0023 (JP)
(74) Representative: Dzieglewska, Hanna Eva
(86) International application number: PCT/JP2005/009299
(87) International publication number: WO 2005/113792

(57) **Abstract**

A probe for detection and quantification with high accuracy in a noninvasive manner as to where and when inositol-1,4,5-trisphosphate is generated in living cells, and a method for detecting and quantifying inositol-1,4,5-trisphosphate using the probe.

## Description

### DESCRIPTION

A Probe for Detection and Quantification of Inositol-1,4,5-trisphosphate and A Method for Detecting and Quantifying Inositol-1,4,5-trisphosphate

### Using the Same

### Technical Field

The invention of this application relates to a probe for spatiotemporal visualization of inositol-1,4,5-trisphosphate in a noninvasive manner, a method for detecting and quantifying ionositol-1,4,5-trisphosphate, a method for monitoring a stimulation effect on generation of inositol-1,4,5-trisphosphate in a cell, and a method for screening a substance inhibiting Ca²⁺ release induced by inositol-1,4,5-trisphosphate by using the probe.

### Background Art

Inositol-1,4,5-trisphosphate (IP₃) is a second messenger in living cells. Stimulations with hormones, growth factors, neurotransmitters, etc. activate phospholipase Cs, which then induces hydrolysis of phosphatidylinositol-4,5-biphosphate (PIP₂) to generate IP₃. It induces release of Ca²+ in a Ca²⁺ store into cytoplasm upon binding to a Ca²⁺ channel, an IP₃ receptor, on a smooth-surfaced endoplasmic reticulum or a sarcoplasmic reticulum membrane.

IP₃ is ubiquitously generated in tissues and organs of almost all animals, and it is one of the most important signaling molecules controlling a large number of biological responses such as fertilization, morphogenesis, angiogenesis and neural functions. Accordingly, it is expected to obtain knowledge on diverse biological responses by detecting where and when IP₃ is generated in living cells.

As a probe to measure the concentration of IP₃ in living cells, a fusion protein of green fluorescent protein (GFP) and pleckstrin homology domain (PH domain) was developed (Non-Patent Document 1). The fusion protein binds to both PIP₂ and IP₃. The probe bound to PIP₂ at the plasma membrane translocates from the plasma membrane to the cytoplasm in response to an increased concentration of IP₃. Simultaneous detection of fluorescence intensities of both the plasma membrane and cytoplasm with the probe can analyze IP₃ generation.

However, the said method has a shortcoming in quantitative capability since measurements might be influenced by a difference in PIP₂ concentration between cells or in shape of cells. It has been further problematic in that since axons and dendrites in neurons are very thin, it is difficult to measure fluorescence intensities in the plasma membrane and cytoplasm separately. Accordingly, a method for spatiotemporal visualization of IP₃ generation with high accuracy in a noninvasive manner has been currently in demand.

Under these circumstances, the invention of this application aims to provide a probe for detecting and quantifying where and when inositol-1,4,5-trisphosphate generates in living cells with high accuracy in a noninvasive manner, and provide a method for detecting and quantifying inositol-1,4,5-trisphosphate using the probe.

### Documents

Non-Patent Document 1: Science, 284, 1527-1530, 1999
Non-Patent Document 2: Proc. Natl. Acad. Sci. USA 77; 7380-7384, 1980

### Disclosure of Invention

For solving the foregoing problems, the invention of this application first provides a probe for detection and quantification of inositol-1,4,5-trisphosphate, which comprises at least,
inositol-1,4,5-trisphosphate recognition site containing an inositol-1,4,5-trisphosphate binding domain of inositol-1,4,5-trisphosphate receptor, and
two reporter sites linked with each end of the inositol-1,4,5-trisphosphate recognition site, of which access imposes fluorescence resonance energy transfer, thereby a signal being detectable.

The invention of this application second provides the probe wherein the two reporter sites are yellow fluorescent protein and cyan fluorescent protein, and third provides the probe wherein the polypeptide leading to conformational change upon specific binding to inositol-1,4,5-trisphosphate is an inositol-1,4,5-trisphosphate binding domain of inositol-1,4,5-trisphosphate receptor.

The invention of this application fourth provides the probe wherein a nuclear localization sequence is linked with any one of the two reporter sites.

The invention of this application fifth provides a method for detecting and quantifying inositol-1,4,5-trisphosphate, which comprises making the probe of any one of foregoing to coexist with inositol-1,4,5-trisphosphate, and measuring signal change with and without inositol-1,4,5-trisphosphate.

The invention of this application sixth provides the method wherein the probe coexists with inositol-1,4,5-trisphosphate in a cell by introducing polynucleotide expressing the probe into the cell, and seventh provides the method wherein probe coexists with inositol-1,4,5-trisphosphate in all cells of a non-human animal or its progeny established by introducing polynucleotide expressing the probe into a non-human totipotent cell and developing the cell to individual.

The invention of this application eighth provides a method for monitoring a stimulation effect on generation of inositol-1,4,5-trisphosphate in a cell, which comprises introducing a polynucleotide expressing the probe of any one of foregoing into the cell, stimulating the cell, and measuring signals before and after the stimulation, and ninth provides a method for monitoring a stimulation effect on generation of inositol-1,4,5-trisphosphate in a living individual, which comprises stimulating a non-human animal or its progeny established by introducing polynucleotide expressing the probe of any one of goregoing into a non-human totipotent cell and developing the cell to individual, and measuring signals before and after the stimulation.

The invention of this application tenth provides a method for screening an inhibitor of Ca²+ release induced by inositol-1,4,5-trisphosphate, which comprises making the probe of the third invention, inositol-1,4,5-trisphosphate and candidate substance to coexist, and measuring signal change with and without the candidate substance.

The invention of this application eleventh provides the screening method, wherein the probe coexists with inositol-1,4,5-trisphosphate and the candidate substance in a cell by introducing polynucleotide expressing the probe into the cell, and twelfth provides the screening method, wherein the probe coexists with inositol-1,4,5-trisphosphate and the candidate substance in all cells of a non-human animal or its progeny established by introducing polynucleotide expressing the probe into a non-human totipotent cell and developing the cell to individual.

The probe of the first to third inventions comprises at least inositol-1,4,5-trisphosphate recognition site and two reporter sites linked with each end of the recognition site, wherein the recognition site contains a polypeptide leading to conformational change upon the specific binding to inositol-1,4,5-trisphosphate thereby the access of two reporters is detectable. When the polypeptide in the recognition site of the probe recognizes inositol-1,4,5-trisphosphate and specifically bind thereto, the conformational change of the polypeptide occurs and the distance and orientation between the two marker sites is changed. Consequently, inositol-1,4,5-trisphosphate can be detected and quantified by measuring the signal change from the marker sites.

In the probe of the fourth invention, a nuclear localization sequence is further linked, whereby the probe can be localized in a nucleus. Accordingly, inositol-1,4,5-trisphosphate generation in the nucleus can be detected and quantified.

In the fifth to seventh inventions, the probe coexists with inositol-1,4,5-trisphosphate and the signal change is measured, whereby inositol-1,4,5-trisphosphate can be detected and quantified easily in a noninvasive manner. For example, in cells or in all cells of a non-human animal or its progeny, the probe can coexist with inositol-1,4,5-trisphosphate.

In the eighth and ninth inventions, a stimulation is applied to a cell into which a polynucleotide expressing the probe is introduced, or a non-human animal or its progeny established by introducing the polynucleotide expressing the probe into the non-human totipotent cell and developing the cell to individual. The stimulation effect on inositol-1,4,5-trisphosphate generation in cells or the living individual can be monitored by measuring the signal change before and after the stimulation.

In the tenth to twelfth inventions, the probe and inositol-1,4,5-trisphosphate coexist, and the signal change is measured with and without a candidate substance. This makes it possible to confirm whether or not the candidate substance inhibits the binding of inositol-1,4,5-trisphosphate with the inositol-1,4,5-trisphosphate receptor and to screen a substance inhibiting Ca²⁺ release induced by inositol-1,4,5-trisphosphate.

### Brief Description of the Drawings

Fig. 1 is a schematic view of the probe for detection and quantification of inositol-1,4,5-trisphosphate in the invention of this application.
Fig. 2 is visualization of ATP-dependent IP₃ generation in MDCK cells using the probe of this invention. (a), 100 µM ATP; (b), 10 µM ATP; and (c), 1 µM ATP.
Fig. 3 is visualization of IP₃ concentration in neural dendrites using the probe of this invention.
Fig. 4 is visualization of IP₃ generation in the MDCK cell nucleus using the probe of this invention.

### Best Mode for Carrying Out the Invention

The probe for detection and quantification of inositol-1,4,5-trisphosphate of this invention comprises at least the following two portions having different functions:
(1) inositol-1,4,5-trisphosphate recognition site which contains a polypeptide specifically binding to inositol-1,4,5-trisphosphate and leading to conformational change upon the binding to inositol-1,4,5-trisphosphate, and
(2) two marker sites linked with each end of the polypeptide, which access each other upon binding of the polypeptide with inositol-1,4,5-trisphosphate and emit a signal. Inositol-1,4,5-trisphosphate is sometimes referred to as "IP₃" in the present specification.

Principle of the probe of this invention is that binding the polypeptide in IP₃ recognition site with IP₃ induces a conformational change of the polypeptide, which then changes the steric configuration of the two reporters linked with each end of the IP₃ recognition site, thereby a signal change is appeared.

In the probe of this invention, IP₃ binding domain on the N terminal side of the IP₃ receptor is preferably exemplified, as the polypeptide leading to its conformational change upon specific binding to IP₃.

IP₃ receptor is a tetrameric complex, and subunit types 1, 2 and 3 are different in IP₃-induced Ca²⁺ release activity and expression distribution in cells and tissues. The IP₃ binding domain as the polypeptide may be any of the IP₃ receptor subunits, types 1 to 3. According to the studies of the present inventors, an especially good response is obtained when using a region from 224th amino acid to 579th amino acid of the type 1 IP₃ receptor as the IP₃ binding domain. Of course, such a polypeptide is not limited to the IP₃ binding domain, and all peptide chains of synthetic or natural can be used.

In the probe of this invention, any reporter sites are available so long as the signal change takes place with high accuracy in response to the conformational change of the polypeptide upon binding to IP₃. Among various fluorescent chromophores in the biochemical field, as those quickly responding to the conformational change, there are chromophores changing fluorescence intensity ratio by occurrence of fluorescence resonance energy transfer (hereinafter referred to as FRET). Accordingly, as the two marker sites, two fluorescent chromophores different in fluorescence wavelength, specifically, a yellow fluorescent protein (YFP), a red-shifted mutant protein of green fluorescent protein (GFP) and a cyan fluorescent protein (CFP), a blue-shifted mutant protein of GFP may be employed. Of course, as the two reporter sites, various fluorescent proteins, split renilla luciferase, firefly luciferase, (β-galactosidase, β-lactamase etc. may be employed.

The probe of this invention may have other sites unless the binding to IP₃ and the access of the two marker sites are inhibited.

For example, in the probe of this invention, a nuclear localization signal sequence (NLS) may be linked with any one of the two reporter sites. NLS includes, for example, a sequence of SEQ ID NO: 1, or known NLS such as a nucleoplasmin-derived sequence (SEQ ID NO: 2) and a HIV-1 Rev-derived sequence (SEQ ID NO: 3), without limitation. The probe is localized in the nucleus, IP₃ transferred into the nucleus can be detected and IP₃ concentration in the nucleus can be quantified.

In order that the two reporter sites are sterically spaced-apart positions without IP₃ and quickly approach upon binding of the polypeptide with IP₃, a linker sequence may be provided between the IP₃ recognition site and each marker site. The nuclear localization sequence and the marker sites may be linked directly or via the linker sequence.

The probe of this invention binds to IP₃ to allow the signal change. Accordingly, making the probe to coexist with IP₃ and measuring the signal change by various chemical or biochemical analysis methods can detect IP₃. IP₃ may be quantified by previously calibrating a relation of fluorescence intensities and IP₃ concentrations.

Moreover, in the invention of this application, using the probe can screen a substance inhibiting IP₃-induced Ca²⁺ release. That is, in a condition of the probe being coexisted with IP₃, the signal change with and without the candidate substance is measured. The candidate substance that inhibits the binding of IP₃ with the probe is judged as IP₃-induced Ca²⁺ release inhibitor.

In the method for IP₃ detection and quantification and the method for screening the IP₃-induced Ca²⁺ release inhibitor, various methods are employed to make the probe to coexist with IP₃ or the candidate substance. For example, the probe and a candidate substance may coexist with IP₃ in elute of disrupted cell.

Alternatively, the probe may coexist with IP₃ in a cell by introducing an expression vector having a polynucleotide for the probe into each culture cell. The candidate substance can be introduced into the cell to make it to coexist with the probe. Such a method enables the IP₃ detection and quantification in vivo without disrupting the cell. As the expression vector, a plasmid vector for animal cells is preferably used. As a method for introducing such a plasmid vector into cells, known methods such as an electroporation method, a calcium phosphate method, a liposome method and a DEAE dextran method may be employed.

In the invention of this application, the probe can coexist with IP₃ in all cells of a non-human animal or its progeny established by introducing a polynucleotide expressing the probe into a non-human totipotent cell and developing the cell to individual in accordance with a known method (for example, Non-Patent Document 2). Since such a transgenic non-human animal carries the probe in all body cells, introducing the candidate substance into the body thereof and measuring the IP₃ concentration in cells or tissues can screen the IP3-induced Ca²⁺ release inhibitor.

In the invention of this application, introducing the polynucleotide expressing the probe into the cell can monitor a stimulation effect on IP₃ generation in a cell, stimulating the cell and measuring the signal change before and after the stimulation. That is, the IP₃ generation amount can be estimated from the signal change of the stimulated cell, and from which the stimulation effect on the IP₃ generation can be known. The stimulation may be biochemical stimulation such as hormones and endocrine disrupters, or physical stimulation such as electricity, radiation, heat etc.

A stimulation effect on IP₃ generation in a living individual and an effect of IP₃ generation on life activities can be also monitored. For this purpose, a non-human animal or its progeny, which is established by introducing the polynucleotide expressing the probe into the non-human totipotent cell and developing the cell to individual, is stimulated, and the signal change before and after the stimulation is measured.

The embodiments of this invention are illustrated more specifically below by referring to Examples along the attached drawings. Of course, this invention is not limited to the following Examples, and it goes without saying that various modifications are possible for details.

### Examples

### Example 1

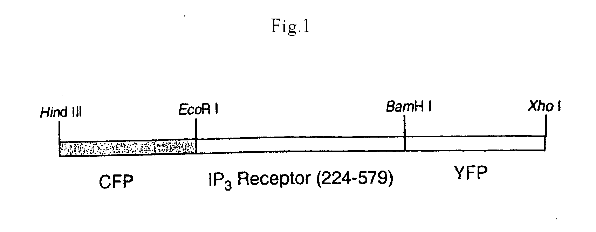

cDNA encoding a probe for IP₃ detection and quantification (Fig. 1) was prepared by a genetic engineering method, and introduced into MDCK cell. Expression of the probe in the cytoplasm was confirmed since fluorescence was observed from the cells with a fluorescence microscope.

### Example 2

Into the MDCK cell expressing the probe as obtained in Example 1, IP₃ was introduced by a microinjection method. On each introduction of a small amount of IP₃, CFP/YFP fluorescence intensity ratio was stepwise increased and saturated soon. It was clarified from this results that the probe recognizes IP₃ and FRET was inhibited dependent on IP₃ concentration.

### Example 3

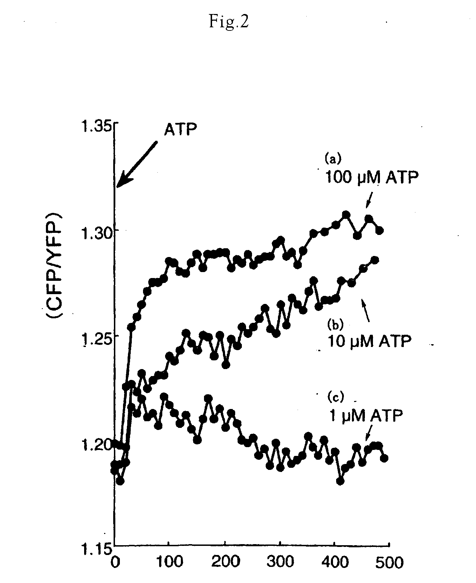

An increase in fluorescence intensity ratio (CFP/YFP) was observed by stimulating a purine receptor of the MDCK cell as obtained in Example 1 with 100 µM ATP (Fig. 2a). Even with 10 µM ATP and 1 µM ATP stimulations, a response of the probe was also observed depend on ATP concentration (Figs. 2b and 2c).
From this results, it has been confirmed that the temporal IP₃ generation in the MDCK cell under physiological conditions can be visualized using the probe of this invention.

### Example 4

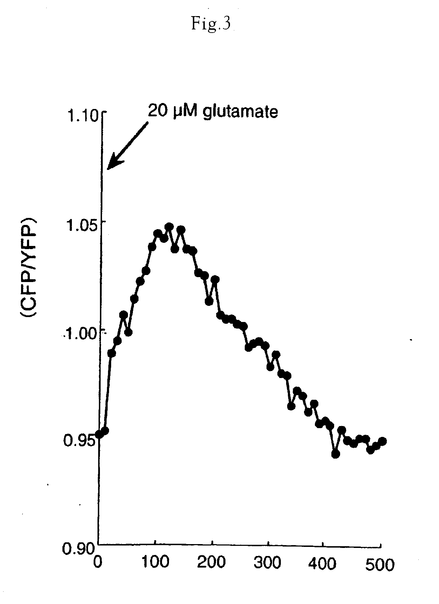

The probe was expressed in a neuron prepared from hippocampus of a rat embryo, and IP₃ kinetics in the neuron was observed.
With 20 µM glutamate stimulation of the neuron, IP₃ concentration was temporally increased in not only the cell body but also the dendrites (Fig. 3).

### Example 5

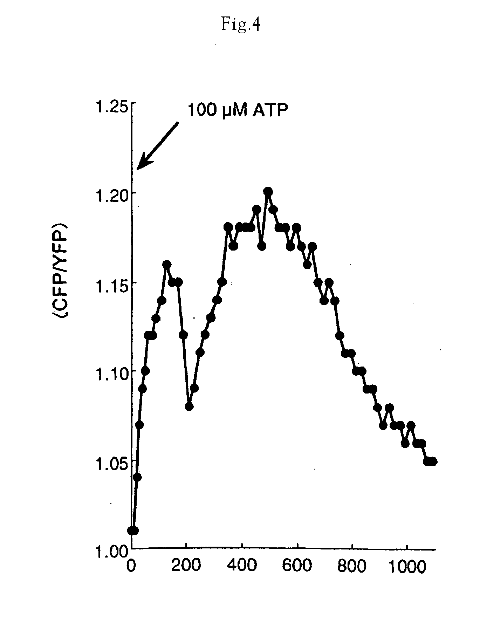

The probe was linked with the nuclear localization sequence of SEQ ID NO: 4 at the C terminal thereof, and expressed in MDCK cell. Since almost all fluorescences were observed from the nucleus, it was confirmed that the probe was localized in the nucleus. With 100 µM ATP stimulation of the MDCK cell, IP₃ concentration was temporally increased (Fig. 4).
It has been reported that there are localized in nucleus an IP₃ receptor and an enzyme group which further phosphorylates IP₃ and produces second messengers such as IP₄, IP₅ and IP₆ having different roles from IP₃. However, it has not been clarified as to what extent IP₃ as a substrate of the enzymes exists in the nucleus, and whether IP₃ can permeate a nuclear pore.
Using the probe of this invention, new knowledge that the IP₃ concentration is increased not only in the cytoplasm but also in the nucleus with extracellular stimulation has been obtained.

### Industrial Applicability

As has been thus far described in detail, this invention provides a probe for detection and quantification with high accuracy in the noninvasive manner as to where and when inositol-1,4,5-trisphosphate is produced in living cells. Further, this invention provides a method for detecting and quantifying inositol-1,4,5-trisphosphate, a method for monitoring a stimulation effect on inositol-1,4,5-trisphosphate generation in the cell and a method for screening a inhibitor for Ca²+ release induced by inositol-1,4,5-trisphosphate, using the probe for detection and quantification of inositol-1,4,5-trisphosphate.

## Claims

1. A probe for detection and quantification of inositol-1,4,5-trisphosphate, which comprises at least,
inositol-1,4,5-trisphosphate recognition site containing an inositol-1,4,5-trisphosphate binding domain of inositol-1,4,5-trisphosphate receptor, and
two reporter sites linked with each end of the inositol-1,4,5-trisphosphate recognition site, of which access imposes fluorescence resonance energy transfer, thereby a signal being detectable.

2. The probe of claim 1, wherein the two reporter sites are yellow fluorescent protein and cyan fluorescent protein.

3. The probe of claim 1 or 2, wherein the polypeptide leading to conformational change upon specific binding to inositol-1,4,5-trisphosphate is an inositol-1,4,5-trisphosphate binding domain of inositol-1,4,5-trisphosphate receptor.

4. The probe of any one of claims 1 to 3, wherein a nuclear localization sequence is linked with any one of the two reporter sites.

5. A method for detection and quantification of inositol-1,4,5-trisphosphate, which comprises making the probe of any one of claims 1 to 4 to coexist with inositol-1,4,5-trisphosphate, and measuring signal change with and without inositol-1,4,5-trisphosphate.

6. The method according to claim 5, wherein the probe coexists with inositol-1,4,5-trisphosphate in a cell by introducing polynucleotide expressing the probe into the cell.

7. The method according to claim 5, wherein the probe coexists with inositol-1,4,5-trisphosphate in all cells of a non-human animal or its progeny established by introducing polynucleotide expressing the probe into a non-human totipotent cell and developing the cell to individual.

8. A method for monitoring a stimulation effect on generation of inositol-1,4,5-trisphosphate in a cell, which comprises introducing a polynucleotide expressing the probe of any one of claims 1 to 4 into the cell, stimulating the cell, and measuring signals before and after the stimulation.

9. A method for monitoring a stimulation effect on generation of inositol-1,4,5-trisphosphate in a living individual, which comprises stimulating a non-human animal or its progeny established by introducing polynucleotide expressing the probe of any one of claims 1 to 4 into a non-human totipotent cell and developing the cell to individual, and measuring signals before and after the stimulation.

10. A method for screening an inhibitor of Ca²⁺ release induced by inositol-1,4,5-trisphosphate, which comprises making the probe of claim 3, inositol-1,4,5-trisphosphate and candidate substance to coexist, and measuring signal change with and without the candidate substance.

11. The method according to claim 10, wherein the probe coexists with inositol-1,4,5-trisphosphate and the candidate substance in a cell by introducing polynucleotide expressing the probe into the cell.

12. The method according to claim 10, wherein the probe coexists with inositol-1,4,5-trisphosphate and the candidate substance in all cells of a non-human animal or its progeny established by introducing polynucleotide expressing the probe into a non-human totipotent cell and developing the cell to individual.
